# EUROPEAN PATENT SPECIFICATION

(11) **EP 2 269 692 B1**
(45) Date of publication and mention of the grant of the patent: **19.09.2012**
(21) Application number: 09734281.0
(22) Date of filing: 24.04.2009
(51) Int. Cl.: A61N 5/06, A61H 23/02

(54) **LASER HAIR-LOSS TREATMENT DEVICE**
LASERGERÄT ZUR BEHANDLUNG VON HAARAUSFALL
DISPOSITIF DE TRAITEMENT AU LASER DE PERTE DES CHEVEUX

(30) Priority: 25.04.2008 KR 20080038833; 23.04.2009 KR 20090035586
(43) Date of publication of application: 05.01.2011
(73) Proprietor: Inix Ltd., Gangwon-do 225-814 (KR); Kim, Tae Hyoung, Hoengseong-gun Gangwon-do 225-814 (KR)
(72) Inventor: KIM, Tae Hyoung, Gangwon-do 225-814 (KR)
(74) Representative: Mitola, Marco
(86) International application number: PCT/KR2009/002169
(87) International publication number: WO 2009/131420

(56) References cited:
- WO-A1-2006/125367
- WO-A2-2006/028461
- KR-A- 20040 012 937
- KR-A- 20060 127 281
- KR-A- 20060 127 281
- KR-A- 20080 068 794
- US-A1- 2002 188 334
- US-A1- 2004 153 131
- US-A1- 2008 004 565

## Description

[Technical Field]

The present invention relates to a laser hair-loss treatment device that treats or prevents hair-loss by simultaneously supplying low-output laser and vibration stimulation to the scalp of a human body, in more detail, a laser hair-loss treatment device that can contribute to growing hairs and preventing hair-loss by activating the hair-root cell of the scalp, by using optical pulse of laser diodes, vibrating elements, and heat-emitting elements, and increase productivity and efficiency in radiation of laser by simplifying the structure.

[Background Art]

In general, although the exact reason for hair-loss is not known, it is known that the hair-loss is because of hereditary factors and influence of male sex hormones and stress. Although the male sex hormones do not cause the hair-loss by themselves, it has been known that the testosterone, a kind of male sex hormones, deteriorates the function of hair papillas and hair follicles and damages the blood vessels, thereby causing hair-loss, by being converted into dihydrotestosterone (DHT) due to a reductase and then coupling with an androgen receptor. Further, there are a variety of reasons, such as lack of nutrition for the hair roots and stress due to dyeing, driving, a permanent.

The hair-loss has been treated in various methods, and recently, a method of radiating low-output laser to the scalp has been widely used, which is known to be able to prevent hair-loss, and treat and prevent diseases of the scalp by stimulating the cells, expanding the blood vessels, activating the hair root cells, and promoting blood circulation, by using the low-output laser.

On the other hand, it has known that the laser used for treating the hair-loss is classified in accordance with the wavelength into semiconductor laser of 830~900nm, CO2 laser of 10600nm, Ar laser of 488~514.6nm, Nd-Yag of laser 532nm, rub laser of 694nm, helium-neon laser of 632nm, and Erbium-Yag laser of 2940nm.

In particular, the low-output laser implies from the red visible light having a wavelength of 600nm or more to an infrared ray having a wavelength of 1400nm, and the magnitude of the output is 100mW ~500mW, and the amount of energy per unit area is 0.05~5.0 W/Cm2. Further, treatment of other skin diseases, using infrared light, has been increasing. That is, light is radiated to the skin of a human body, the photon penetrates into the skin tissue, and the absorbed photo is converted into thermal energy, such that thermal energy produces a thermal effect. In particular, it has been known that nitric oxides are created and activated on the inner wall of the blood vessels and self-healing power increases, such that the hair tone and quality are improved and the hairs become moist due to water supplement. Further, it has been known that creation of collagen and elastin is increased by stimulating the giant cells and the fibroblasts, the oxygen in the blood stream increases and toxins are removed, such that the hair-loss is prevented and there is an effect on health of the hairs.

The wavelength of infrared rays used for the treatment method is a blue diode (blue light emitting diode): 400~700nm, a green diode (green light emitting diode):470~572nm, a yellow diode (yellow light emitting diode) :572~630nm, a red diode (red light emitting diode):630~700nm, and a infrared diode (infrared light emitting diode):700~1,400nm etc.

In particular, since the treatment method using the infrared rays is simple and has little side effects, the operation range is gradually increasing. Further, recently, a method that can acquire infrared rays and low-output laser from a semiconductor diode has been developed, such that the size of optical treatment devices is reduced and the devices can be easily used, and accordingly, they are used as products for home or portable products.

However, laser hair-loss treatment devices of the related art uses only the laser from a laser diode for treatment, such that not only the treatment effect is low, but the manufacturing cost is high because the assembly of the laser diode is complicated. Further, replacement and maintenance are difficult.
The patent applications KR20060127281A and WO 2006/028461A2 disclose devices for treating the scalp with a combination of light and vibration sources.

[Disclosure]

[Technical Problem]

The present invention has been made to overcome the problems of the related art and it is an object of the present invention to provide a laser hair-loss treatment device that makes it possible to simply assemble a laser diode to facilitate assembly/disassembly and maintenance, and can improve activation of the hair root cells of in the scalp by simultaneously applying optical pulse of a laser diode, physical stimulation, and thermal stimulation.

Further, it is another object of the present invention to provide a laser hair-loss treatment that can maintain comfortable wearing sensation by disposing laser optical cables in the inner skin of a cap such that the laser optical cables can directly contact the user's scalp, to minimize laser loss and simplify the structure of the inner skin of the cap.

[Technical Solution]

In order to achieve the object of the present invention, a laser hair-loss treatment device according to an embodiment of the present invention includes a cap covering the user's scalp and a laser assembly disposed in the cap and radiating laser by receiving power by control of a controller, in which the laser hair-loss treatment device includes: an elastic pad that is attached to the inner side of the cap; a circuit board that is attached to one side of the elastic pad and having a heat-emitting body generating heat by receiving power; vibration motors that are electrically connected to the circuit board without interfering the position of the heat-emitting body, and vibrate by receiving power; and a laser assembly that is disposed at one side of the vibration motor, electrically connected to the circuit board, and has one end protruding through an assembly hole formed through the elastic pad.

According to a preferred aspect of the present invention, the laser assembly further includes: a lens that receives a laser diode generating laser light by receiving power and collects/diffuses the laser light; a housing that has a space for receiving the lens and an open side for the top of the lens to protrude; and a lens cap that closes the open side of the housing, has a through hole for the top of the lens to protrude, and has one end thread-fastened to the housing.

According to a preferred aspect of the present invention, the elastic pad is made of any one of silicon, urethane, rubber, and synthetic resin.

According to the present invention, the heat-emitting body is any one of a wire type heat-emitting body or a surface type heat-emitting body that generates heat by receiving power in response to a control signal from the controller.

According to a preferred aspect of the present invention, the housing further includes a heat-dissipating plate at the portion where the laser diode is mounted.

A laser hair-loss treatment device according to another embodiment of the present invention includes a cap covering the user's scalp and a laser assembly disposed in the cap and radiating laser by receiving power by control of a controller, in which the laser hair-loss treatment device further includes: an elastic pad that is attached to the inner side of the cap; a circuit board that is attached to one side of the elastic pad and having a heat-emitting body generating heat by receiving power; vibration motors that are electrically connected to the circuit board without interfering the position of the heat-emitting body, and vibrate by receiving power; and a laser assembly that includes a laser oscillator that is disposed at on side of the cap and generates laser in response to a control signal from the controller and a lens that is connected with the laser oscillator by an optical cable, receives the end of the optical cable, and has one end protruding through an assembly hole formed through the elastic pad to contact the user's scalp.

According to a preferred aspect of the present invention, the laser assembly further includes: the lens that receives the end of the optical cable connected to the laser oscillator to transmit laser light and collects/diffuses the laser light; a housing that has a space for receiving the lens and one side open for the top of the lens to protrude; and a lens cap that has a through-hole such that the top of the lens can protrude, and is thread-fastened in the housing.

According to a preferred aspect of the present invention, the elastic pad is made of any one of silicon, urethane, rubber, and synthetic resin.

According to the present invention, the heat-emitting body is any one of a wire type heat-emitting body or a surface type heat-emitting body that generates heat by receiving power in response to a control signal from the controller.

According to a preferred aspect of the present invention, the housing further includes a heat-dissipating plate at the portion where the laser diode is mounted.

A laser hair-loss treatment device according to another embodiment of the present invention includes a cap radiating laser to the user's scalp, in which the laser hair-loss treatment device includes: a casing that is disposed at one side of the cap and receives one or a plurality of laser diodes generating laser light by receiving power; a condensing lens that is installed at one side of the laser diode and collects laser radiated; a connecting pipe that includes a bunch of optical fibers and has one end connected to one side of the casing in order to transmit the laser passing through the condenser lens to one side; an elastic pad that is attached to the inner side of the cap and connected with the other end of the connecting pipe such that the optical fibers disposed therein; and brush hairs that protrude from one side of the elastic pad to contact the user's scalp and each have one end connected to the fibers to radiate laser.

According to a preferred aspect of the present invention, the casing includes: a circuit board that has a circuit composed of the laser diode and electronic devices; and a heat-dissipating plate that is disposed at one side of the circuit board and dissipates heat generated from the laser diode.

According to a preferred aspect of the present invention, the elastic pad is made of any one of silicon, urethane, rubber, and synthetic resin.

According to a preferred aspect of the present invention, ring blocks each having a hole at the center are fitted in the elastic pad, and the ends of the brush hairs are inserted in the holes of the ring blocks.

According to a preferred aspect of the present invention, one or more condenser lenses are disposed at a predetermined distance.

According to a preferred aspect of the present invention, the elastic pad includes vibration motors 50 that vibrate by receiving power without interfering with the positions of the brush hairs.

Features and advantages of the present invention will be made clear from the following detailed description based on the accompanying drawings. Terms or words used in the specification and claims herein should be not construed as a general and lexical meaning and should be construed as the meaning and concept meeting the technical idea of the present invention based on a principle that the present inventors can properly define the concepts of terms in order to elucidate their own invention in the best method.

[Advantageous Effects]

A laser hair-loss treatment device according to the present invention can activate the hair root cells in various ways by applying optical pulse stimulation using laser diodes, physical stimulation using vibrating elements, and thermal stimulation using heat-emitting elements, to the scalp of a wearer, such that it is expected to promote growing hairs and preventing hair-loss.

Further, it is possible to improve workability in assembling and disassembling by simplifying the configuration of a laser diode assembly, and it is possible to promote penetration of laser and medicine, because laser and vibration are transmitted, with a lens and a lens cap in contact with the scalp.

Further, the present invention is expected to advantages of minimizing laser loss and simplifying the structure of the inner skin of the cap by disposing laser optical cables in the inner skin of the cap and directly contacting the laser optical cable to the user's scalp, and of satisfying consumers' demand for the product by maintaining good wearing sensation and improving usability.

Although preferred embodiments of the present invention were described above, it should be understood that the present invention may be changed and modified in various ways by those skilled in the art, without departing from the scope and the spirit of the present invention described in claims.

[Description of Drawings]

FIG. 1 is a view illustrating when a laser hair-loss treatment device according to an embodiment of the present invention is used;

FIG. 2 is an exploded perspective view showing the main parts of the laser hair-loss treatment device according to an embodiment of the present invention;

FIG. 3 is a perspective view showing an assembly of the parts shown in FIG. 2;

FIG. 4 is a cross-sectional view showing the main parts of the laser hair-loss treatment device according to an embodiment of the present invention;

FIG. 5 is a view illustrating when a laser hair-loss treatment device according to another embodiment of the present invention is used;

FIG. 6 is a perspective view illustrating the configuration of the main parts shown in FIG. 5;

FIG. 7 is a cross-sectional view showing the main parts of a laser hair-loss treatment device according to another embodiment of the present invention;

FIG. 8 is a view illustrating when the laser hair-loss treatment device according to the present invention is used;

FIG. 9 is a perspective view illustrating the configuration of the laser hair-loss treatment device according to the present invention;

FIG.10 is a cross-sectional view schematically illustrating the configuration of the laser hair-loss treatment device according to the present invention; and

FIG. 11 is a cross-sectional view showing the main parts of the laser hair-loss treatment device according to the present invention.

<Reference Numerals>

| | | | |
|---|---|---|---|
| 1: | Laser hair-loss treatment device | | |
| 10: | Cap | 20: | Circuit board |
| 30: | Elastic pad | 40: | Laser assembly |
| 50: | Vibration motor | 70: | Laser generator |

[Best Mode]

Objects, features, and advantages of the present invention, which were described above, will be clear from the following detailed description.

Hereinafter, preferred embodiments of the present invention are described with reference to the accompanying drawings. It should be noted that the same components and parts are designated by the same reference numerals in the drawings. It should be understood that the detailed description for related well-known functions and configurations is not provided in order not to make the spirit of the present invention unclear in describing the present invention.

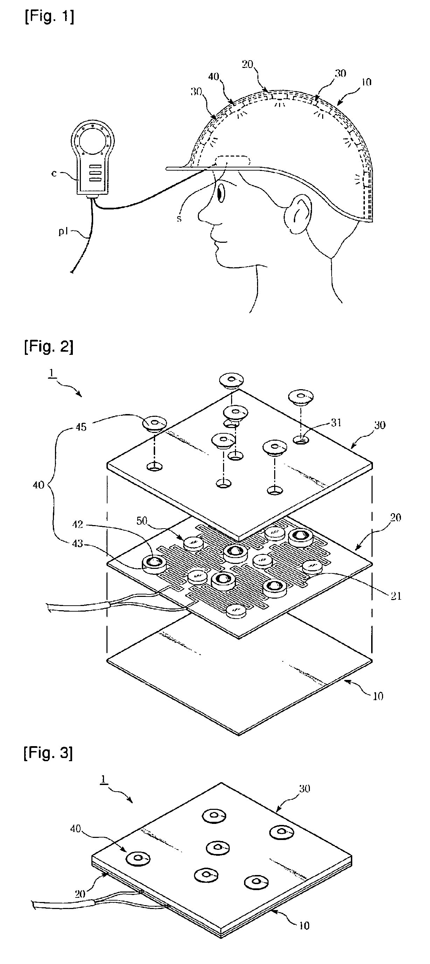

FIG. 1 is a view illustrating when a laser hair-loss treatment device according to an embodiment of the present invention is used, FIG. 2 is an exploded perspective view showing the main parts of the laser hair-loss treatment device according to an embodiment of the present invention, and FIG. 3 is a perspective view showing an assembly of the parts shown in FIG. 2.

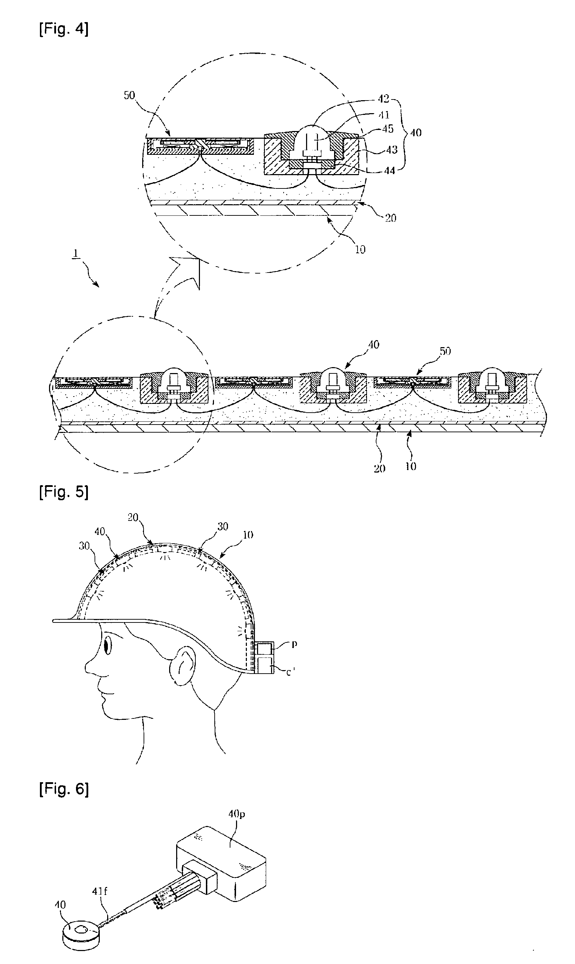

Further, FIG. 4 is a cross-sectional view showing the main parts of the laser hair-loss treatment device according to an embodiment of the present invention.

As shown in the figures, a laser hair-loss treatment device of the present invention includes: a cap 10 that covers the user's head, such as a headgear; a controller 'c' with operation buttons and a display window which is connected to a side of the cap 10 by a wire or in wireless and controls electronic devices in response to operational signals from the user; a sensor `s' that is attached to a predetermined portion on the inner side of the cap 10 to sense temperature and whether a user wears the cap, etc.; an elastic pad 30 that is attached to the inner side of the cap 10; a circuit board 20 that is positioned between the elastic pad 30 and the cap 10; vibration motors 50 that are mounted on the circuit board 20 and vibrate; and a laser assemblies 40 that are electrically connected to the circuit board 20 and each have one end formed in pressing protrusion shape that presses the user's head, and radiates laser light.

The cap 10 is put on the user's head and may be implemented in any shapes known in the art, such that the detailed description is not provided.

The elastic pad 30 disposed in the cap 10 is made of an elastic material having a predetermined thickness, and preferably, made of silicon, urethane, rubber, or synthetic resin to be easily machined, improve wearing sensation, and easily remove foreign substances.

The elastic pad 30, as shown in the figures, has assembly holes 31 at regular intervals such that lenses 42 of the laser assembly 40 (described blow) can partially protrude through them.

The circuit board 20 is attached to one side of the elastic pad 30 and positioned between the elastic pad 30 and the cap 10 in the figures to be selectively supplied with power in response to control signals from the controller 'c', through a circuit. It is preferable that the circuit board 20 is a soft circuit board and a heat-emitting body 21 is selectively provided at one side. In this configuration, the heat-emitting body 21 is a wire type heat-emitting body or a surface type heat-emitting element. Reference numeral "pl' that was not stated above indicates a power line connected to the controller 'c'.

The vibration motors 50, elements stimulating the scalp by applying predetermined vibration to the user's head, vibrate by selectively receiving power in response to a control signal from the controller 'c'. It is preferable to use a slim coin-typed vibration motors for the vibration motors 50 and the coin-typed vibration motors has been well known in the art, such that the detailed description is not provided.

The laser assemblies 40, elements radiating laser to the user's scalp, are electrically connected to the circuit board 20 without interfering with the positions of the vibration motors 50 and the heat-emitting body 21. One end of the laser assembly 40 protrudes through the assembly hole 31 formed through the elastic pad 30 and the protruding portion contacts the user's scalp.

The laser assembly 40 includes a lens 42 having a laser diode 41 generating laser light by receiving power in response to a control signal from the controller 'c', in which the lens 42 may be a concave or convex lens that can collect or diffuse laser light.

Further, the lens 42 is accommodated in a housing with one side open and a portion of the lens 42 protrudes through the open side of the housing 43.

Meanwhile, the open side of the housing 43 is closed by a lens cap 45 and the lens cap 45 has a through-hole (not given a reference numeral) such that a portion of the lens 42 can protrude outside. The lens cap 45 has the lower portion extending downward to be thread-fastened to the inner side of the housing, when seen in the figures.

Further, the housing 43 has a heat-dissipating plate 44 where the laser diode 41 is disposed.

The laser assembly 40 having the above configuration allows laser light radiated from the laser diode 41 to travels to the user's scalp through the lens 42 at the open side of the housing 43, and the laser diode 41 is covered by the housing 43 and the heat-dissipating plate 44, except for the side through which the laser light is radiated, such that heat generated from the laser diode 41 is discharged through the open side of the housing 43 and thermal stimulation is applied to the user's scalp.

Use of the laser hair-loss treatment device having the above configuration according to the present invention is described hereafter.

First, a user operates the operation button in the controller 'c', with the cap 10 on the head, to turn on the device. The sensor `s' disposed at a predetermined portion on the inner side of the cap 10 senses whether the device is worn and power is supplied to the vibration motor 50, the heat-emitting body 21, and the laser diode 41 of the laser assembly 40, which are electrically connected to the circuit board 20.

Accordingly, the heat-emitting body 21 generates heat at predetermined temperature and uniformly heats the entire elastic pad 30, while the vibration motor 50 vibrates and the laser diode 41 radiates laser light to the user's scalp.

In this process, since the laser diode 41, as shown in the figures, protrudes from the elastic pad 30, it is in contact or positioned close to the user's scalp, such that it radiates laser light from a close position and also physically contacts the scalp by the vibration of the vibration motor 50. Further, the laser diode 41 is covered by the housing 43 and the heat-dissipating plate 44, except for one side, heat generated from the laser diode 41 is transmitted to the scalp to apply thermal stimulation to the scalp.

FIG. 4 is a cross-sectional view showing the main parts of the laser hair-loss treatment device according to an embodiment of the present invention and FIG. 5 is a view illustrating when a laser hair-loss treatment device according to another embodiment of the present invention is used.

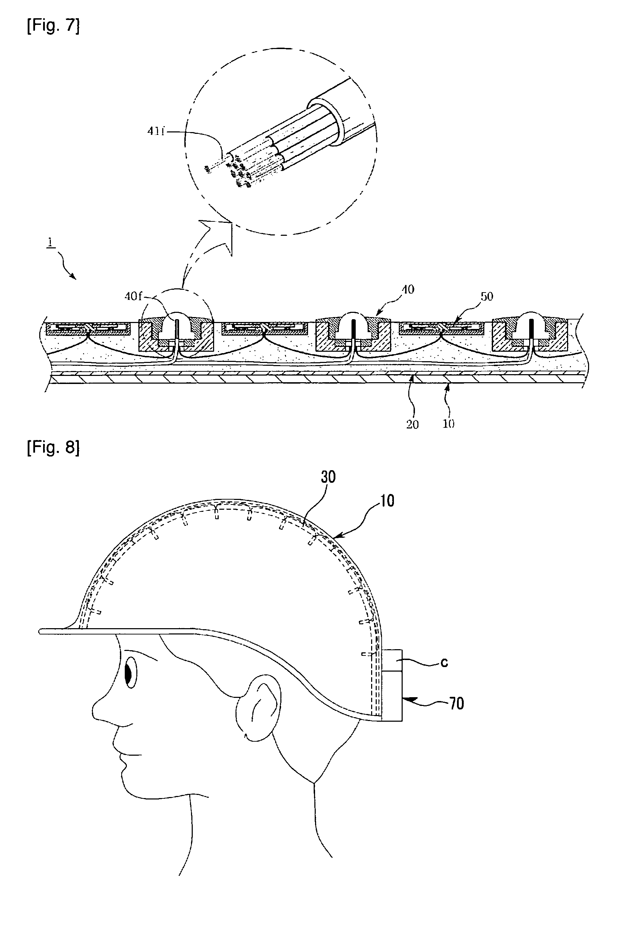

Further, FIG. 6 is a perspective view illustrating the configuration of the main parts shown in FIG. 5 FIG. 7 is a cross-sectional view showing the main parts of a laser hair-loss treatment device according to another embodiment of the present invention.

As shown in the figures, the laser hair-loss treatment device of this embodiment has substantially the same configuration as the embodiment described above. However, this embodiment is **characterized in that** the laser diode is replaced by a laser oscillator 40p, and a plurality of optical cables 41f are connected to divide the laser light radiated from the laser oscillator 40p, thereby implementing a laser assembly 40 with the optical cables 41f.

That is, the laser hair-loss treatment device 1 according to this embodiment, as in the embodiment described above, includes a cap 10 that covers the user's head, such as a headgear; a controller 'c' that is connected to a side of the cap 10 by a wire or in wireless and controls electronic devices in response to operational signals from the user; a sensor `s' that is attached to a predetermined portion on the inner side of the cap 10 to sense temperature and whether a user wears the cap, etc.; an elastic pad 30 that is attached to the inner side of the cap 10; a circuit board 20 that is positioned between the elastic pad 30 and the cap 10; vibration motors 50 that are mounted on the circuit board 20 and vibrate; and a laser assemblies 40 that are electrically connected to the circuit board 20 and each have one end formed in pressing protrusion shape that presses the user's head, and radiates laser light.

The cap 10, elastic pad 30, circuit board 20, and vibration motors 50 are the same as those of the embodiment described above, and the detailed description is not provided.

In this embodiment, the laser assembly 40 is characterized by being composed of the laser oscillator 40p that is disposed at on side of the cap 10 and generates laser in response to a control signal from the controller 'c' and a lens 42 that is connected with the laser oscillator 40p by the optical cable 41f, receives the end of the optical cable 41f, and has one end protruding through an assembly hole 31 formed through the elastic pad 30 to contact the user's scalp.

That is, the laser assembly 40 includes the lens 42 that receives the end of the optical cable 41f connected to the laser oscillator 40p to transmit laser light and collects/diffuses the laser light, a housing 43 that has a space for receiving the lens 42 and one side open for the top of the lens 42 to protrude, and a lens cap 45 that has a through-hole such that the top of the lens 42 can protrude, and is thread-fastened in the housing 43.

The laser hair-loss treatment device 1 having the above configuration according to another embodiment of the present invention is the same in use as the embodiment described above, and the detailed description is not provided.

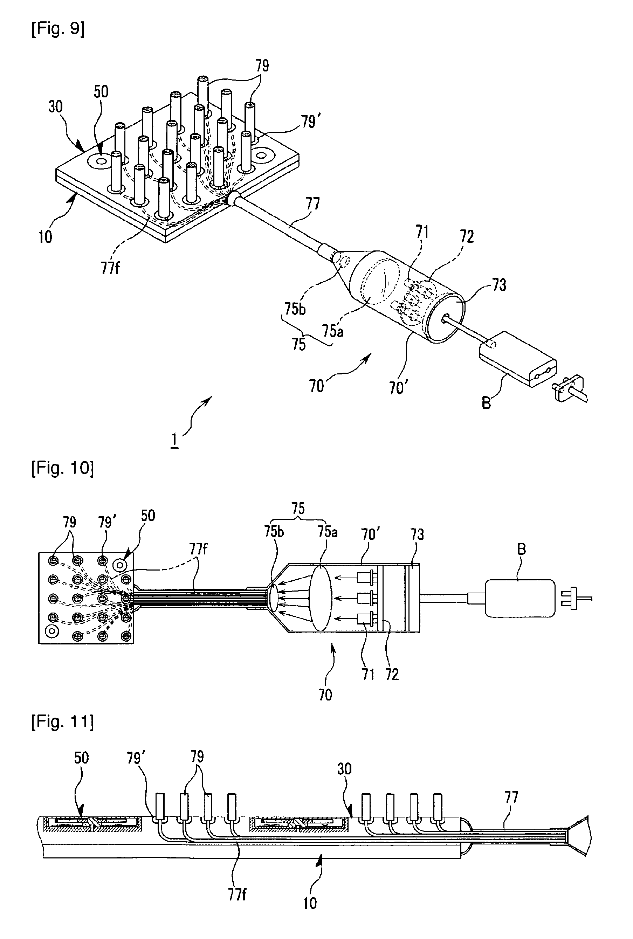

The laser hair-loss treatment device according to another embodiment of the present invention is described with reference to FIGS. 8 to 11. FIG. 8 is a view illustrating when the laser hair-loss treatment device according to the present invention is used, FIG. 9 is a perspective view illustrating the configuration of the laser hair-loss treatment device according to the present invention, FIG. 10 is a cross-sectional view schematically illustrating the configuration of the laser hair-loss treatment device according to the present invention, and FIG. 11 is a cross-sectional view showing the main parts of the laser hair-loss treatment device according to the present invention.

As shown in the figures, the laser hair-loss treatment device 1 according to the present invention includes: a cap 10 that covers the user's head, such as a headgear; a controller 'c' with operation buttons and a display window which is connected to a side of the cap 10 by a wire or in wireless and controls electronic devices in response to operational signals from the user; an elastic pad 30 that is attached to the inner side of the cap 10; vibration motors 50 that are vibrated by power supplied from the outside to pressing the user's scalp; and brush hairs 79 that protrude without interfering with the positions of the vibration motors 50 and are made of optical fibers to transmit laser generated from the laser generator 70 to the user's scalp.

The cap 10 is put on the user's head and may be implemented in any shapes known in the art, such that the detailed description is not provided.

The elastic pad 30 disposed in the cap 10 is made of an elastic material having a predetermined thickness, and preferably, made of silicon, urethane, rubber, or synthetic resin to be easily machined, improve wearing sensation, and easily remove foreign substances. The elastic pad 30, as shown in the figures, is equipped with the brush hairs 79 and the vibration motors 50 at regular intervals.

The vibration motors 50, elements stimulating the scalp by applying predetermined vibration to the user's head, vibrate by selectively receiving power in response to a control signal from the controller 'c'. It is preferable to use a slim coin-typed vibration motors for the vibration motors 50 and the coin-typed vibration motors has been well known in the art, such that the detailed description is not provided.

The laser generator 70, an element that generates laser by receiving power, is composed of a casing 70' that forms the outer shape, a plurality of laser diode 71 that is disposed in the casing 70' and generates laser by receiving power, a condensing lens 75 that collects laser radiated from the laser diodes 71, and a connecting pipe 77 that includes a bunch of optical fibers 77f that transmit the laser collected through the condenser lens 75.

The casing 70' is positioned not to detract wearing sensation when the user wears the cap 10 and includes the laser diodes 71 that generates laser by receiving power from the outside. The laser diodes 71 are mounted in a circuit on a circuit board 72, and various electronic devices, other than the laser diodes 71, are mounted in the circuit on the circuit board 72. It is preferable to use a soft circuit board for the circuit board 72. Further, a heat-dissipating plate 73 made of metal having high thermal conductivity may be additionally provided at one side of the circuit board 72 to dissipate heat. The heat-dissipating plate 73 dissipates heat generated from the laser diodes 71.

The laser diodes 71 are diodes that generate laser by using forward semiconductor conjunction, which is implemented by well-known technologies, and the detailed description is not provided. One or a plurality of laser diodes 71 is provided in the present invention, as shown in the figures, and the laser diodes are arranged in the circuit on the circuit board 72 such that the laser is radiated in the same direction.

The condenser lens 75 is an element disposed ahead of the laser diodes 71, that is, on the path of the laser radiated to collect the laser. The condenser lens 75 may be a concave or a convex lens, which are different, and one lens may be provided or two or more lenses having different collection magnifications may be disposed at a predetermined distance.

The connecting pipe 77 includes the optical fibers 79 connected to the brush hairs 79 vertically protruding from one side of the elastic pad 30 to transmit the laser light passing through the condenser lens 75.

That is, a bunch of optical fibers 77f are disposed in the connecting pipe 77 and the optical fibers 77f are disposed inside the elastic pad 30 through the connecting pipe 77. Further, laser light can be consequently radiated to the user's scalp by connecting the optical fibers disposed in the elastic pad 30 to the brush hairs 79.

The laser generator 70 having the above configuration generates laser light, using the laser diodes 71, by receiving power, and the laser light generated in this way is collected through the condenser lens 75. Further, the collected laser light is transmitted to the brush hairs 79 through the optical fibers 77f disposed in the elastic pad 30 through the connecting pipe 77.

The brush hairs 79 are optical fibers vertically protruding from one side of the elastic pad 30 to be able to contact the user's scalp and connected to the optical fibers disposed in the elastic pad 30 through the connecting pipe 77 to allow the transmitted laser light to be radiated through the ends.

The brush hairs 79 are arranged in a lattice shape to be able to contact the user's scalp at regular intervals, as shown in the figures, and the vibration motors 50 are disposed between the brush hairs 79 without interfering with the positions.

Further, the brush hairs 79 is fitted in ring blocks 79' to be firmly fixed to the elastic pad 30, in which the ring blocks 79' each have a hole at the center such that one end of the brush hairs 79 can pass through the hole. The ring block 79' has a diameter larger than the brush hair 79 to occupy a large area and is fixed to the elastic pad 30 by bonding or sewing.

A reference numeral 'b', which was not stated, indicates a chargeable battery, which is charged by being selectively connected to a constant power source and implemented by well-known technologies, and the detailed description is not provided.

The operation of the laser hair-loss device having the above configuration according to the present invention is described hereafter.

First, a user turns on the treatment device by operating a button in the controller 'c', with the cap 10 on the head.

Power is supplied to the vibration motors 50 on the elastic pad 30, which is the inner skin of the cap 10, and the laser generator 70, such that the vibration motors 50 apply stimulation to the user's scalp by vibrating and the laser generator 70 generates and radiates laser light to the user's scalp through the brush hairs 79.

In this operation, since the laser light radiated from the laser diodes 71 of the laser generator 70 is transmitted to the brush hairs 79 through the condenser lens 75 and the connecting pipe 77 and the brush hairs 79 protrude from the elastic pad 30 that is the inner skin of the cap 10, the laser light is radiated from a close position through the ends contacting or positioned close to the user's scalp.

Meanwhile, the present invention is not limited to the embodiment described above and it is apparent to those skilled in the art that the present invention may be changed and modified in various ways without departing from the scope of the present invention. Therefore, the changes and modifications are construed as being included in claims of the present invention.

## Claims

1. A laser hair-loss treatment device that includes a cap (10) covering the user's scalp and a laser assembly disposed in the cap (10) and radiating laser by receiving power by control of a controller (c), the laser hair-loss treatment device further comprising:
an elastic pad (30) that is attached to the inner side of the cap (10);
a circuit board (20) that is attached to one side of the elastic pad (30) and having a circuit composed of electronic devices;
vibration motors (50) that are electrically connected to the circuit board (20) and vibrate by receiving power; and
a laser assembly (40) that includes a laser diode (41) electrically connected to the circuit board (20) and selectively radiating laser light and a lens (42) covering the laser diodes (41) and having one end protruding through an assembly hole (31) formed through the elastic pad (30) to contact the user's scalp;
said device being **characterized in that** the circuit board (20) further includes a heat-emitting body (21) that selectively generates heat by receiving power, and
the heat-emitting body (21) is any one of a wire type heat-emitting body and a surface type heat-emitting body that generates heat by receiving power in response to a control signal from the controller (c).

2. The laser hair-loss treatment device according to claim 1, wherein the laser assembly (40) further includes:
the lens (42) that receives the laser diode (41) generating laser light by receiving power and collects/diffuses the laser light;
a housing (43) that has a space for receiving the lens (42) and an open side for the top of the lens (42) to protrude; and
a lens cap (45) that closes the open side of the housing (43), has a through hole for the top of the lens (42) to protrude, and has one end thread-fastened to the housing (43).

3. The laser hair-loss treatment device according to claim 1 or 2, wherein the elastic pad (30) is made of any one of silicon, urethane, rubber, and synthetic resin.

4. The laser hair-loss treatment device according to claim 2, wherein the housing (43) further includes a heat-dissipating plate (44) at the portion where the laser diode (41) is mounted.

## Patentansprüche

1. Laser-Haarausfallbehandlungsvorrichtung, die eine Kappe bzw. Haube (10), die den Kopf bzw. die Kopfhaut eines Benutzers bedeckt, und eine Laseran-ordnung enthält, die in der Kappe bzw. Haube (10) angeordnet ist und Laser ausstrahlt, indem sie Leistung durch Steuerung bzw. Regelung einer Steuer- bzw. Regelsyeinrichtung (c) empfängt, wobei die La-ser-Haarausfallbehandlungsvorrichtung ferner umfasst:
ein elastisches Kissen bzw. Pad (30), das an der Innenseite der Kappe bzw. Haube (10) angebracht ist;
eine Leiterplatte (20), die an einer Seite des elastischen Kissens bzw. Pads (30) angebracht ist und eine Schaltung aufweist, die aus elektronischen Vorrichtungen besteht;
Vibrationsmotoren (50), die elektrisch mit der Leiterplatte (20) verbunden sind und vibrieren, indem sie Leistung empfangen; und
eine Laseranordnung (40), die eine Laserdiode (41), die elektrisch mit der Leiterplatte (20) verbunden ist und selektiv Laserlicht ausstrahlt, und eine Linse (42) enthält, welche die Laserdioden (41) abdeckt und bei der ein Ende durch ein Anordnungsloch (31) ragt, das durch das elastische Kissen bzw. Pad (30) gebildet ist, um den Kopf bzw. die Kopfhaut des Benutzers zu kon-taktieren;
wobei die Vorrichtung **dadurch gekennzeichnet ist, dass** die Leiterplatte (20) ferner einen Wärme aussendenden Körper (21) enthält, der selektiv Wärme erzeugt, indem er Leistung empfängt, und
der Wärme aussendende Körper (21) einer eines Wärme aussendenden Kör-pers vom Drahttyp und eines Wärme aussendenden Körpers vom Oberflä-chentyp ist, der Wärme erzeugt, indem er Leistung empfängt, und zwar ansprechend auf ein Steuer- bzw. Regelsignal von der Steuer- bzw. Regelein-richtung (c).

2. Laser-Haarausfallbehandlungsvorrichtung nach Anspruch 1, wobei die Laseranordnung (40) ferner enthält:
die Linse (42), welche die Laserdiode (41) empfängt bzw. aufnimmt, die Laserlicht erzeugt, indem sie Leistung empfängt, und das Laserlicht sam-melt/streut;
ein Gehäuse (43), das einen Raum zum Empfangen bzw. Aufnehmen der Linse (42) und eine offene Seite für die Oberseite der Linse (42) zum Vorra-gen aufweist; und
eine Linsenkappe bzw. -haube (45), welche die offene Seite des Gehäuses (43) schließt, die ein Durchgangsloch für die Oberseite der Linse (42) zum Vorragen aufweist und die ein Ende aufweist, das an dem Gehäuse (43) gewindebefestigt ist.

3. Laser-Haarausfallbehandlungsvorrichtung nach Anspruch 1 oder 2, wobei das elastische Kissen (30) aus einem von Silizium bzw. Silikon, Urethan, Gummi und Kunstharz besteht.

4. Laser-Haarausfallbehandlungsvorrichtung nach Anspruch 2, wobei das Ge-häuse (43) ferner eine wärmeableitende Platte (44) an dem Abschnitt enthält, wo die Laserdiode (41) montiert ist.

## Revendications

1. Dispositif de traitement au laser de la perte de cheveux comprenant une coiffe (10) qui recouvre le crâne de l'utilisateur, et un ensemble laser qui est disposé dans la coiffe (10) et qui émet des rayons laser lorsqu'il reçoit du courant suite à une commande d'un dispositif de commande (c), le dispositif de traitement au laser de la perte de cheveux comprenant en outre :
un coussin élastique (30) qui est attaché au côté intérieur de la coiffe (10) ;
une carte à circuit imprimé (20) qui est attachée à un côté du coussin élastique (30) et qui présente un circuit imprimé composé de dispositifs électroniques ;
des moteurs à vibration (50) qui sont électriquement connectés à la carte à circuit imprimé (20) et qui vibrent lorsqu'ils reçoivent du courant ; et
un ensemble laser (40) comprenant une diode laser (41) qui est électriquement connectée à la carte à circuit imprimé (20) et qui émet de façon sélective des rayons de lumière laser, et une lentille (42) qui couvre les diodes laser (41) et dont une extrémité fait saillie à travers un trou d'assemblage (31) qui est formé à travers le coussin élastique (30) pour entrer en contact avec le crâne de l'utilisateur ;
ledit dispositif étant **caractérisé en ce que** la carte à circuit imprimé (20) comprend en outre un corps d'émission de chaleur (21) qui génère de la chaleur de façon sélective lorsqu'il reçoit du courant ; et
le corps d'émission de chaleur (21) est soit un corps d'émission de chaleur du type par fil, soit un corps d'émission de chaleur du type par surface, qui génère de la chaleur en recevant du courant en réponse à un signal de commande envoyé par le dispositif de commande (c).

2. Dispositif de traitement au laser de la perte de cheveux selon la revendication 1, dans lequel l'ensemble laser (40) comprend en outre :
la lentille (42) qui reçoit la diode laser (41) qui génère la lumière laser en recevant du courant et qui collecte/diffuse la lumière laser ;
un boîtier (43) qui présente un espace destiné à recevoir la lentille (42), et un côté ouvert à travers lequel le sommet de la lentille (42) fait saillie ; et une coiffe de lentille (45) qui ferme le côté ouvert du boîtier (43), qui comporte un trou traversant à travers lequel le sommet de la lentille (42) fait saillie ; et qui présente une extrémité attachée par vissage au boîtier (43).

3. Dispositif de traitement au laser de la perte de cheveux selon la revendication 1 ou 2, dans lequel le coussin élastique (30) est constitué soit de silicone, soit d'uréthane, soit de caoutchouc, soit d'une résine synthétique.

4. Dispositif de traitement au laser de la perte de cheveux selon la revendication 2, dans lequel le boîtier (43) comprend en outre une plaque de dissipation de chaleur (44) qui est située à la partie à laquelle la diode laser (41) est montée.
